(19) Europäisches Patentamt
European Patent Office
Office européen des brevets

(11) **EP 2 272 424 A1**

(12) **EUROPEAN PATENT APPLICATION**

(43) Date of publication:
**12.01.2011 Bulletin 2011/02**

(51) Int Cl.:
*A61B 5/00* (2006.01)
*G01B 9/02* (2006.01)
*G01N 21/47* (2006.01)

(21) Application number: **10185570.8**

(22) Date of filing: **31.10.2005**

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.10.2004 US 623773 P**

(62) Document number(s) of the earlier application(s) in accordance with Art. 76 EPC:
**05821139.2 / 1 819 270**

(71) Applicant: **The General Hospital Corporation Boston, MA 02114 (US)**

(72) Inventors:
• **Park, Boris Hyle**
**Somerville, MA 02143 (US)**

• **DeBoer, Johannes F.**
**Somerville, MA 02145 (US)**

(74) Representative: **Lawrence, John**
**Barker Brettell LLP**
**100 Hagley Road**
**Edgbaston**
**Birmingham**
**B16 8QQ (GB)**

Remarks:
This application was filed on 01-10-2010 as a divisional application to the application mentioned under INID code 62.

(54) **Polarisation-sensitive optical coherence tomography**

(57) A polarisation-sensitive optical coherence tomography (OCT) system is disclosed comprising a frequency-swept light source (100) and a polarisation modulating arrangement (115). Preferably, the system comprises fibre-optic components. Also disclosed is a Jones matrix based analysis approach which compensates for polarisation effects created by the fibre-optic components of the OCT system.

FIG.1

EP 2 272 424 A1

**Description**

STATEMENT OF FEDERAL SUPPORT

This invention was made, at least in part, with Government support under grant numbers R01EY014975, and R01RR19768 from the National Institute of Health, and grant numbers F49620-01-10014 and FA-9550-04-1-0079 from the Department of Defense. The Government may have certain rights to the invention described and claimed herein.

CROSS-REFERENCE TO RELATED APPLICATION(S)

This application is based upon and claims the benefit of priority from U.S. Patent Application Serial No. 60/623,773, filed October 29, 2004, the entire disclosure of which is incorporated herein by reference.

FIELD OF THE INVENTION

The present invention relates to systems and methods for a fiber-based optical imaging using a low coherence light beam reflected from a sample surface and combined with reference light beam, in which an evolution of the polarization state of the sample arm light can be used to determine the polarization parameters of the sample.

BACKGROUND OF THE INVENTION

Optical coherence tomography is an imaging technique that measures the interference between a reference beam of light and a beam reflected back from a sample. A detailed system description of traditional time-domain OCT was first described in Huang et al. "Optical Coherence Tomography," Science 254, 1178 (1991). Detailed system descriptions for spectral-domain OCT and Optical Frequency Domain Interferometry are given in International Patent Application No. PCT/US03/02349 and U.S. Patent Application No. 60/514,769, respectively. Polarization-sensitive OCT provides additional contrast by observing changes in the polarization state of reflected light. The first fiber-based implementation of polarization-sensitive time-domain OCT was described in Saxer et al., "High-speed fiber-based polarization-sensitive optical coherence tomography of in vivo human skin," Opt. Lett. 25, 1355 (2000).

In polarization-sensitive time-domain OCT, simultaneous detection of interference fringes in two orthogonal polarization channels allows complete characterization of the reflected polarization state as described in J.F. de Boer et al., "Determination of the depth-resolved Stokes parameters of light backscattered from turbid media by use of polarization-sensitive optical coherence tomography," Opt. Lett. 24, 300 (1999). There are two non-depolarizing polarization parameters: birefringence, characterized by a degree of phase retardation and an optic axis orientation, and diattenuation, which is related to dichroism and characterized by an amount and an optic axis orientation. There are two generally accepted and approximately equivalent formalisms for characterizing polarization states: using complex orthogonal electric fields and Jones matrices, and using Stokes vectors and Mueller matrices. A review of the evolution of Stokes parameters as a function of depth has been used to characterize polarization properties such as birefringence and optic axis orientation in a variety of biological samples as described in the Saxer publication and B. Cense et al., "In vivo depth-resolved birefringence measurements of the human retinal nerve fiber layer by polarization-sensitive optical coherence tomography," Opt. Lett. 27, 1610 (2002).

The polarization state reflected from the sample can be compared to the state incident on the sample quite easily in a bulk optic system, as the polarization state incident on the sample can be controlled and fixed. However, an optical fiber has a disadvantage of that propagation through optical fiber can alter the polarization state of light. In this case, the polarization state of light incident on the sample is not easily controlled or determined. Also, the polarization state reflected from the sample is not necessarily the same as that received at the detectors. Assuming negligible diattenuation, or polarization-dependent loss, optical fiber changes the polarization states of light passing through it in such a manner as to preserve the relative orientation between states. The overall effect of propagation through optical fiber and non-diattenuating fiber components is similar to an overall coordinate transformation or some arbitrary rotation. In other words, the relative orientation of polarization states at all points throughout propagation is preserved as described in U.S. Patent No. 6,208,415.

There have been a number of approaches that take advantage of this fact to determine the polarization properties of a biological sample imaged with polarization-sensitive OCT. However, all of these techniques are disadvantageous in some manner. A vector-based method has been used to characterize birefringence and optic axis orientation only by analyzing rotations of polarization states reflected from the surface and from some depth for two incident polarization states perpendicular in a Poincaré sphere representation as described in the Saxer Publication, J.F. de Boer et al., "Determination of the depth-resolved Stokes parameters of light backscattered from turbid media by use of polarization-sensitive optical coherence tomography," Opt. Lett. 24, 300 (1999), and B.H. Park et al., "In vivo burn depth determination by high-speed fiber-based polarization sensitive optical coherence tomography," J. Biomed. Opt. 6, 474 (2001). Mueller matrix based methods are capable of determining birefringence, diattenuation, and optic axis orientation as described in S.L. Jiao et al., "Two-dimensional depth-resolved Mueller matrix of biological tissue measured with double-beam polarization-sensitive optical coherence tomography," Opt. Lett. 27, 101 (2002), S. Jiao et al., "Optical-fiber-based Mueller optical coherence tomography," Opt. Lett. 28, 1206 (2003), and S.L. Jiao et al., "Depth-resolved two-dimensional Stokes vectors of backscattered light and Mueller matrices of biological tissue measured with optical coherence tomography," Appl. Opt, 39, 6318 (2000).

These techniques typically invoke a multitude of measurements using a combination of incident states and detector settings and limits their practical use for in vivo imaging. Jones matrix based approaches have also been used to fully characterize the non-depolarizing polarization properties of a sample as described in S. Jiao et al., "Optical-fiber-based Mueller optical coherence tomography," Opt. Lett. 28, 1206 (2003) and S.L. Jiao and L.V. Wang, "Jones-matrix imaging of biological tissues with quadruple-channel optical coherence tomography," J. Biomed. Opt, 7, 350 (2002). The description of these approaches has limited the use of optical fiber and fiber components such as circulators and fiber splitters such that these components must be traversed in a round-trip fashion. A methodology that allows a determination of non-depolarizing polarization parameters with the unrestricted use of such components in an optical imaging system may be desirable.

SUMMARY OF THE INVENTION

According to the present invention, exemplary systems, software arrangements and processes are provided for determining the non-depolarizing polarization properties of a sample imaged by OCT with no restrictions on the use of optical fiber or non-diattenuating fiber components, such as circulators and splitters. These properties include, but are not limited to, cumulative and differential phase retardation, cumulative and differential diattenuation, and cumulative and differential optic axis orientation.

The exemplary embodiments of the process, software arrangement and system according to the present invention are capable of characterizing the amount and orientation of the axis of birefringence, assuming little or no diattenuation, between two locations of a sample imaged by OCT with no restrictions on the use of optical fiber or non-diattenuating fiber components. In addition, it is possible to characterize the amount and orientation of diattenuation, assuming little or no birefringence, between two locations of a sample imaged by OCT with no restrictions on the use of optical fiber or non-diattenuating fiber components.

In addition, the exemplary embodiments of the process, software arrangement and system according to the present invention can be used to determine the non-depolarizing polarization properties of a sample by comparing the light reflected from two different locations within the sample probed with a minimum of two unique incident polarization states in such a way that allows for the unrestricted use of optical fiber and non-diattenuating fiber components throughout the system.

Thus, according to the exemplary embodiments of the present invention, it is possible to:

a. determine the full polarization properties of a sample, including but not limited to phase retardation, diattenuation, and optic axis orientation, probed with a minimum of two unique incident polarization states,

b. use two incident polarization states approximately perpendicular in a Poincaré sphere representation to set up optimal detection of sample polarization properties including birefringence,

c. provide an unrestricted placement of optical fiber and non-diattenuating fiber components throughout a polarization-sensitive OCT system,

d. determine the overall Jones matrix and its corresponding polarization parameters of interest by optimization of general functions of complex electric fields, including but not limited to magnitude, phase, and polynomial, logarithmic/ exponential, and trigonometric combinations thereof, and

e. modify these optimization procedures and the resulting parameter determination to better match the physical situation.

According an exemplary embodiment of the present invention, arrangement, system and method for a polarization effect for a interferometric signal received from sample in an optical coherence tomography ("OCT") system are provided. In particular, an interferometric information associated with the sample and a reference can be received. The interferometric information is then processed thereby reducing a polarization effect created by a detection section of the OCT system on the interferometric signal. Then, an amount of a diattenuation of the sample may be determined. The interferometric information can be provided at least partially along at least one optical fiber which can be provided in optical communication with and upstream from a polarization separating arrangement.

In another exemplary embodiment of the present invention, at least one polarization property of the sample can be determined. The polarization property may include a depolarizing property, a birefringence property, an optic axis of the polarization property, and/or further information associated with at least two polarization states incident on the sample. The interferometric information can be processed by:

a. determining a first state of one of the polarization states at a first location at least one of within or in a proximity of the sample,
b. determining a second state of another one of the polarization states at least one of at or near the first location at least one of within or in the proximity of the sample,
c. determining a third state of one of the polarization states at a second location at least one of within or in the proximity of the sample,
d. determining a fourth state of another one of the polarization states at least one of at or near the second location at least one of within or in the proximity of the sample,
e. generating a complex 2x2 matrix so as to transform the first and second states into the third and fourth states, respectively, and
f. decompose the matrix into a product of further matrixes, wherein first and second of the further matrices are unitary and inverse of one another, and selected to minimize off-diagonal elements of a third one of the further matrices.

For example, at least two of the first through fourth states which are obtained at locations that are at least one of the same as or different from the first and second locations are averaged.

According to another exemplary embodiment of the present invention, apparatus and method are provided for transmitting electromagnetic radiation to a sample. For example, at least one first arrangement can be provided which is configured to provide at least one first electro-magnetic radiation. A frequency of radiation provided by the first arrangement can vary over time. At least one polarization modulating second arrangement can be provided which is configured to control a polarization state of at least one first electro-magnetic radiation so as to produce at least one second electro-magnetic radiation. Further, at least one third arrangement can be provided which is configured to receive the second electromagnetic radiation, and provide at least one third electro-magnetic radiation to the sample and at least one fourth electro-magnetic radiation to a reference. The third and fourth electro-magnetic radiations may be associated with the second electromagnetic radiation.

In still another exemplary embodiment of the present invention, at least one fifth electro-magnetic radiation can be provided from the sample, and at least one sixth electro-magnetic radiation may be provided from the reference. The fifth and sixth electro-magnetic radiations are associated with the third and fourth electromagnetic radiations, respectively. In addition, at least one fourth arrangement can be provided which is configured to receive at least one seventh electro-tromagnetic radiation which is associated with the fifth and sixth electro-magnetic radiations, and produce at least one eighth electromagnetic radiation having a first polarization state and at least one ninth electromagnetic radiation a second polarization state based on the seventh electromagnetic radiation. The first and second polarization states are preferably different from one another.

At least one fifth arrangement can be provided which is configured to receive and/or detect the eighth and ninth electromagnetic radiations, and determine an amplitude and/or a phase of the eighth and/or ninth electromagnetic radiations. In addition or alternatively, the fifth arrangement can receive and/or detect the eighth and ninth electromagnetic radiations, receive and/or detect at least one tenth radiation associated with the first, second, fourth and/or sixth electro-tromagnetic radiations, thereby reducing noise associated with fluctuations of the first electromagnetic radiation and/or the second electromagnetic radiation. Further, the fifth arrangement is capable of determining the amplitude and/or the phase of the eighth electromagnetic radiation and/or the electromagnetic radiation.

According to still another exemplary embodiment of the present invention, polarization states associated with the fifth electromagnetic radiation can be determined at different depth in the sample and/or a proximity of the sample as a function of the amplitude and/or the phase of the eighth and/or ninth electromagnetic radiations, and based on the second electro-magnetic radiation. At least one of first through ninth electromagnetic radiations can be propagated via at least one optical fiber.

Pursuant to a further exemplary embodiment of the present invention, at least one ophthalmic imagine sixth arrangement can be provided which is configured to received the third electromagnetic radiation, and produce the fifth electromagnetic radiation. A processing arrangement can be provided, which when executing a predetermined technique, can be configured to receive data associated with the amplitude and/or the phase of the eighth and/or ninth electromagnetic radiations, and process the data thereby reducing a polarization effect created by at least one portion of the apparatus (e.g., OCT system) on the seventh electromagnetic radiation, and determining polarization properties of the sample. The polarization properties can include birefringence, diattenuation, depolarization, optic axis of the birefringence, and/or optic axis of the diattenuation.

These and other objects, features and advantages of the present invention will become apparent upon reading the following detailed description of embodiments of the invention, when taken in conjunction with the appended claims.

BRIEF DESCRIPTION OF THE DRAWINGS

Further objects, features and advantages of the invention will become apparent from the following detailed description taken in conjunction with the accompanying figures showing illustrative embodiments of the invention, in which:

Fig. 1 is a schematic diagram of an exemplary embodiment of a fiber-based polarization-sensitive time-domain OCT system which is and/or can be used with the exemplary systems, software arrangements and processes according to the present invention;

Fig. 2 is a plot of an output of a polarization sensitive optical coherence tomography ("PS-OCT")-derived relative optic axis orientation of a polarizing sheet as a function of its true orientation, wherein inset can be the same optic axes plotted on a Poincaré sphere;

Fig. 3 is a plot of single-pass phase retardation as a function of depth obtained using the exemplary systems, software arrangements and processes according to the present invention;

Fig. 4 is a plot of single-pass diattenuation as a function of depth obtained using the exemplary systems, software arrangements and processes according to the present invention; and

Fig. 5 is a flow diagram of an exemplary embodiment of a method according to the present invention.

DETAILED DESCRIPION

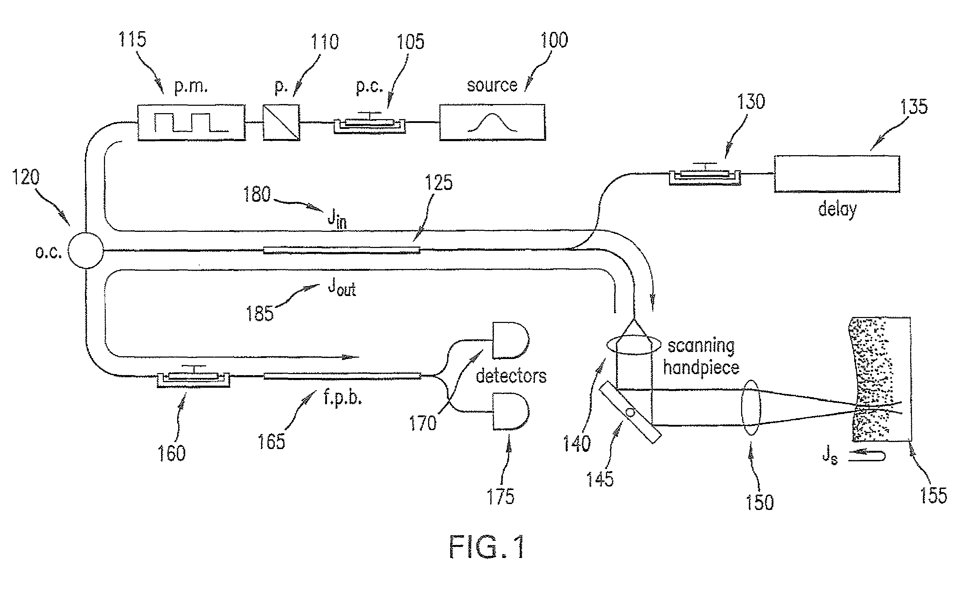

The exemplary embodiments of systems, software arrangements and processes can be implemented in a variety of OCT or OFDI systems. Fig. 1 shows an exemplary embodiment of a fiber-based polarization-sensitive time-domain OCT arrangement which is and/or that can be used for implementing the exemplary embodiments of the system, process, and software arrangement according to the present invention.

The exemplary embodiments of the method, system and arrangement according to the present invention can be implemented in a variety of imaging systems. For example, as shown in Fig. 1, the exemplary arrangement which is and/or may be used with exemplary embodiments of the present invention is provided with components of an exemplary fiber-based OCT system, and a standard single-mode fiber may be used throughout such arrangement. In particular, the arrangement includes a light (e.g., broadband) source 100 which is adapted to generate an electromagnetic radiation or light signal. A polarization controller 105 and a polarizer 110 can be included, and may be used to select a polarization state that has, e.g., the highest power of the light source 100. This light and/or electromagnetic radiation can be transmitted to an electro-optic polarization modulator 115 which is configured based on a two-step driving function that is adapted to switch or toggle the polarization state between two orthogonal states in a Poincaré sphere representation.

After passing through an optical circulator 120 that is provided in the exemplary arrangement, the light/electromagnetic radiation may be separated and transmitted to the sample arm (which includes a sample 155) and the reference arm of the interferometer via a 90/10 fiber splitter 125. A polarization controller 130 may be provided in the reference arm (which includes a reference/delay line 135), and can be used to control the arrangement such that a constant amount of power associated with the light/electromagnetic radiation is transmitted and reflected from the delay line 135. For example, this can be done regardless of the polarization state of the light/electromagnetic radiation in the source arm. The sample arm can be composed of a collimating lens 140, a scanning mechanism 145, and a lens 150 that focuses the beam into the sample 155. The light/electromagnetic radiation returning from both the sample and reference arms then passes back through the fiber splitter 125 and the optical circulator 120 before passing through a polarization controller 160, and then split by a polarization separating element 165. The resulting two sets of interference fringes from the split signals are measured by separate detectors 170, 175.

For example, the optical path from the source to the sample can be represented by a Jones matrix $\mathbf{J_{in}}$ 180, and the optical path from the sample to the detectors can be represented by $\mathbf{J_{out}}$ 185. In particular, $\mathbf{J_{in}}$, $\mathbf{J_{out}}$, and $\mathbf{J_S}$ are the Jones matrix representations for the one-way optical path from the polarization modulator to the scanning hand-piece, the one-way optical path back from the scanning hand-piece to the detectors 170, 175, and the round-trip path through some depth in the sample 155, respectively. In this manner, the exemplary embodiment of the present invention can be used in interferometric imaging systems. According to one further exemplary embodiment of the present invention, the optical circulator 120 and the splitter 125 can be replaced by a single fiber coupler.

This exemplary arrangement can be used in a time-domain OCT configuration, a spectral-domain OCT configuration, an OFDI configuration, and other similar configuration. For example, in the time-domain OCT configuration, the source 100 can be a broadband source, the delay line is capable of scanning over a range, the polarization separating element 165 can be a fiber-polarizing beam splitter, and the detectors 170, 175 can be photodiodes. For the exemplary spectral-domain OCT configuration, the source 100 can be a broadband source, the delay line 135 may be of a fixed length, the polarization separating element 165 can be a polarizing beam splitter cube, and the detectors 170, 175 can be line scan cameras in a spectrometer. In the exemplary OFDI configuration, the source 100 may be a swept source, the delay line 135 can have a fixed length, the polarization separating element 165 can be a fiber-polarizing beam splitter, and the detectors 180, 175 may be photodiodes.

In general, the exemplary embodiments of the system, arrangement and process according to the present invention which are provided for analyzing the polarization properties of electromagnetic radiation can be applied to any apparatus or arrangement that is configured to determine the electric fields reflected from or transmitted through a sample by interfering the sample arm light with a reference. For example, the electric fields may be determined in approximately orthogonal polarized channels by use of a polarization sensitive splitter, that more than one polarization state is used to probe the sample, and that this information is acquired for more than one wavelength in parallel or consecutively at approximately the same sample location. The above described general preferences can be implemented by detection methods known in the art such as but not restricted to time domain optical coherence tomography as described above and also in N.A. Nassif et al., "In vivo human retinal imaging by ultrahigh-speed spectral domain optical coherence tomography," Opt. Lett. 29, 480 (2004), Spectral Domain or Fourier Domain OCT described in the PCT patent application identified above in the Nassif Publication, and Optical Frequency Domain Imaging or Swept Source OCT which was also described above in the identified patent provisional patent application and S.H. Yun et al., "High-speed optical frequency-domain imaging," Opt. Exp. 11, 2953 (2003).

The non-depolarizing polarization properties of an optical system according to an exemplary embodiment of the present invention can be described by its complex Jones matrix, $\mathbf{J}$. This matrix can transform an incident polarization state, described by a complex electric field vector, $\overline{E} = [H\ V]^T$, to a transmitted state, $\overline{E}' = [H'\ V']^T$, and can be decomposed in the form $\mathbf{J} = \mathbf{J_R}\mathbf{J_P} = \mathbf{J_P}\cdot\mathbf{J_{R'}}$, where $\mathbf{J_R}$ and $\mathbf{J_P}$ are the Jones matrices for a retarder and a polarizer, respectively as described in J.J. Gil et al., "Obtainment of the polarizing and retardation parameters of a non-depolarizing optical system from the polar decomposition of its Mueller matrix," Optik 76, 67 (1987). Birefringence, described by $\mathbf{J_R}$, can be parameterized by 3 variables: a degree of phase retardation, $\eta$, about an axis defined by two angles, $\gamma$ and $\delta$. Diattenuation, described by $\mathbf{J_p}$, is defined as $d = \left(P_1^2 - P_2^2\right)/\left(P_1^2 + P_2^2\right)$ and can be parameterized by 4 variables, where $P_1$ and $P_2$ are the attenuation coefficients parallel and orthogonal to an axis defined by angles $\Gamma$ and $\Delta$. These independent parameters, along with an overall common phase $e^{i\psi}$, can account for the 4 complex elements of a general Jones matrix $\mathbf{J}$. Based on the assumption that the birefringence and diattenuation in biological tissue share a common axis ($\delta = \Delta$ and $\gamma = \Gamma$) as described in S.L. Jiao et al., "Two-dimensional depth-resolved Mueller matrix of biological tissue measured with double-beam polarization-sensitive optical coherence tomography," Opt. Lett. 27, 101 (2002), the number of independent parameters can be reduced by two.

An incident and reflected polarization state can yield, e.g., three relations involving the two orthogonal amplitudes and the relative phase between them as described in J.F. de Boer et al., "Determination of the depth-resolved Stokes parameters of light backscattered from turbid media by use of polarization-sensitive optical coherence tomography," Opt. Lett. 24, 300 (1999). Therefore, it is possible to use the six relationships defined by two unique pairs of incident and reflected states to exactly solve for the above Jones matrix. Thus, by probing a sample with only two unique incident states, it is possible to extract all the polarization parameters of interest.

One exemplary implementation of the method, system and arrangement for determining the polarization parameters of interest can be provided as follows. A single computer or a plurality of computers linked together can be used to alternate the polarization state incident on the sample between two states perpendicular in a Poincaré sphere representation for successive depth scans. Assuming negligible diattenuation, the optical paths from the polarization modulator to the sample surface, described by $\mathbf{J_{in}}$, and from the sample surface to the detectors, $\mathbf{J_{out}}$, may be modeled as elliptical retarders. If the electric field after the polarization modulator is defined as $E_{in}$, then the electric field of detected light reflected from the surface of a sample is given by $E = e^{i\psi}\mathbf{J_{out}}\mathbf{J_{in}}E_{in}$. The round-trip Jones matrix of the sample as $\mathbf{J_S}$ can be defined, and the detected light reflected from within the sample may be given by

$$\overline{E'} = e^{i\psi'}\mathbf{J_{out}}\mathbf{J_s}\mathbf{J_{in}}\overrightarrow{E_{in}} = e^{i\Delta\psi}\mathbf{J_{out}}\mathbf{J_s}\mathbf{J_{out}^{-1}}\overrightarrow{E}, \text{where } \Delta\psi = \psi' - \psi.$$ Since the Jones matrices for elliptical retarders are unitary and thus form a closed group, it is possible to rewrite the combined Jones matrix

$$\mathbf{J_T} = \mathbf{J_{out}}\mathbf{J_s}\mathbf{J_{out}^{-1}} = \mathbf{J_U}\begin{bmatrix} P_1 e^{i\eta/2} & 0; 0 & P_2 e^{-i\eta/2} \end{bmatrix}\mathbf{J_U^{-1}}, \text{using}$$

$\mathbf{J_U} = e^{i\beta}[C_0 e^{i(\phi-\varphi)} -S_0 e^{i(\phi-i-\varphi)}; S_0 e^{-i(\phi+\varphi)} C_0 e^{-i(\phi-\varphi)}]$ to describe a general unitary transformation where $C_0 = \cos\theta$ and $S_\theta = \sin\theta$.

It is possible to obtain an alternative formulation for $\mathbf{J_T}$ by combining information from two unique incident states, $\begin{bmatrix} H_1' & H_2'; V_1' & V_2' \end{bmatrix} = e^{i\Delta\psi_1}\mathbf{J_T}\begin{bmatrix} H_1 & e^{i\alpha}H_2; V_1 & e^{i\alpha}V_2 \end{bmatrix}$, where $\alpha = \Delta\psi_2 - \Delta\psi_1$. The polarization parameters of interest can be obtained by equating the two expressions for $\mathbf{J_T}$ to yield

$$e^{i\Delta\psi_1}\begin{bmatrix} P_1 e^{\eta_1/2} & 0 \\ 0 & P_2 e^{-\eta_1/2} \end{bmatrix} = \begin{bmatrix} C_\theta & S_\theta \\ -S_\theta & C_\theta \end{bmatrix}\begin{bmatrix} e^{-i\phi} & 0 \\ 0 & e^{i\phi} \end{bmatrix}\begin{bmatrix} H_1' & H_2' \\ V_1' & V_2' \end{bmatrix}\begin{bmatrix} H_1 & e^{i\alpha}H_2 \\ V_1 & e^{i\alpha}V_2 \end{bmatrix}^{-1}\begin{bmatrix} e^{i\phi} & 0 \\ 0 & e^{-i\phi} \end{bmatrix}\begin{bmatrix} C_\theta & -S_\theta \\ S_\theta & C_\theta \end{bmatrix} \quad (1)$$

The formulation in Eq. 1 is advantageous over conventional methods for extracting polarization parameters of interest.

First, all the polarization parameters of interest may be related to one another in a way that allows for simultaneous determination. In contrast, the conventional vector-based approach mentioned above requires that the optic axis be fully determined before two separate calculations of phase retardation for the two incident polarization states described in B.H. Park et al., "In vivo burn depth determination by high-speed fiber-based polarization sensitive optical coherence tomography," J. Biomed. Opt. 6, 474 (2001). The overall phase retardation can then be taken as a weighted average of the two values as described in B.H. Park et al., "Real-time multi-functional optical coherence tomography," Opt. Exp. 11, 782 (2003).

Second, the formulation in Eq. 1 can be exactly solvable; in other words, the formulation may not lead to under- or over-determination, where there are too few or too many independent equations compared to the number of independent variables. In previous Mueller matrix based analysis methods, there are more available equations when compared to the number of independent polarization parameters.

Third, the formulation and technique according to the exemplary embodiment of the present invention has no requirements on the transpose symmetry of $J_T$. A previous Jones matrix-based analysis for obtaining the full polarization parameters of a sample with fiber-based PS-OCT imposed the condition that the round trip Jones matrix for light returning from the sample surface be transpose symmetric as described in S. Jiao et al., "Optical-fiber-based Mueller optical coherence tomography," Opt. Lett. 28, 1206 (2003).

This procedure generally addresses the assumption that any fiber optic components may be traversed in a round-trip manner to cancel any inherent circular birefringence, to insure $\delta = \Delta = 0$, and achieve transpose symmetry. This prior art procedure restricts the placement of optical fiber and requires a bulk beam splitter in the interferometer instead of a fiber optic splitter. In the formulation and techniques according to the exemplary embodiment of the present invention, transpose symmetry of the overall Jones matrix is not required, thus enabling the use of non-diattenuating fiber optic components, such as splitters and circulators, as well as removing any restrictions on the use of fiber throughout the system. Finally, the formulation of Eq. 1 performs the measurement using only two unique incident polarization states for full determination of the polarization parameters of interest.

In principle, the parameters $\theta$, $\phi$, and $\alpha$ can be solved for with the condition that the off-diagonal elements of the matrix product on the right hand side of Eq. 1 are equal to zero. In practice, real solutions may not always be found, as measurement noise can induce non-physical transformations between incident and transmitted polarization states. To account for this, it is possible to optimize parameters $\alpha$, $\phi$, and $\theta$ to minimize the sum of the magnitudes of the off-diagonal elements. A relative optic axis can be derived from $\phi$ and $\theta$, given in Stokes parameter form by $A = [1\ C_{2\theta}\ S_{2\theta}C_{2\phi}\ S_{2\theta}S_{2\phi}]^T$. The degree of phase retardation can easily be extracted through the phase difference of the resulting diagonal elements, and the diattenuation by their magnitudes. The error on the calculation can be estimated by taking the ratio of the sum of the magnitudes of these off-diagonal elements to the sum of the magnitudes of the diagonal elements. These resulting diattenuation, birefringence, and optic axis orientation values can be differentiated to yield local values for the polarization parameters of interest.

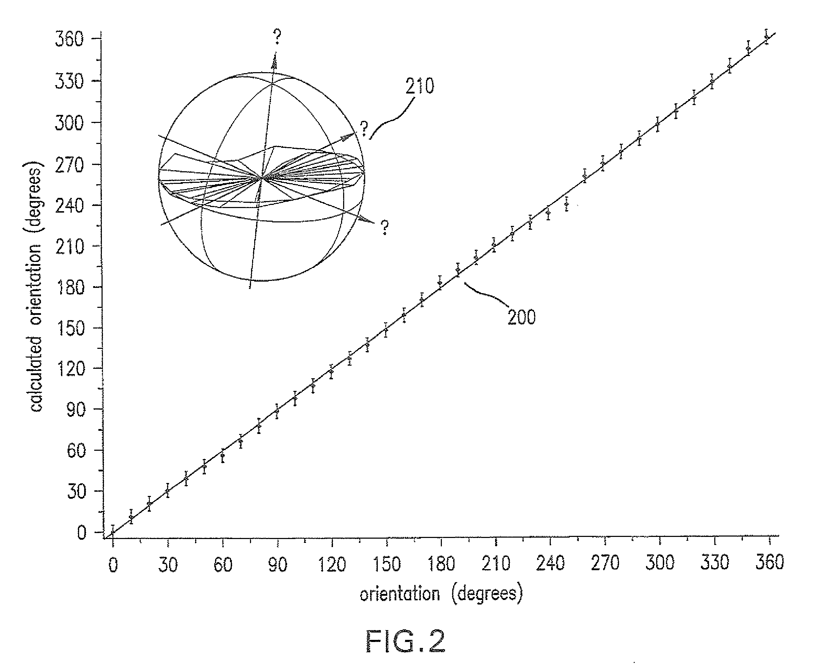

Fig. 2 is a plot of an output of a polarization sensitive optical coherence tomography ("PS-OCT")-derived relative optic axis orientation of a polarizing sheet described above as a function of its true orientation based on the information obtained using a system, arrangement and method in accordance with the present invention, in which inset can be the same optic axes plotted on a Poincaré sphere. In particular, the exemplary images provided by such PS-OCT system were taken of an IR polarizing sheet, orthogonal to the axis of the incident beam, and rotated in 10° increments about this axis, spanning a full 360°. An average single-pass diattenuation value derived from the scans of 0.992±0.002 approximately agrees with an independent measurement of 0.996±0.001, determined by transmission of linearly polarized light, parallel and orthogonal to the optic axis of the sheet. The optic axis determination is shown in Fig. 2, which illustrates the optic axis orientation with respect to the set orientation 200 of the polarizing sheet. The inset 210 provided in the graph illustrates that the optic axes are nearly co-planar and span two full circles on the Poincaré sphere, in agreement with the imaging geometry. The rotation of the plane of optic axes away from the QU-plane is evident as well.

As described, above, $J_T$ can be determined experimentally by using two unique incident polarization states to probe the same volume of a sample. The relationship between these states is important; two nearly identical incident polarization states will work mathematically, but do not truly take advantage of the information provided by two sets of data over just one. An equally important consideration arises from when an incident state becomes aligned with the optic axis of the sample due to fiber birefringence. In this case, the incident and reflected polarization states are identical, and contain no information regarding birefringence. The same will hold for an orthogonal incident polarization state. It becomes clear that while diattenuation can always be determined using two orthogonal incident polarization states, birefringence cannot. A better choice is to use two incident polarization states perpendicular in a Poincaré sphere representation, as previously presented in C.E. Saxer et al., "High-speed fiber-based polarization-sensitive optical coherence tomography of in vivo human skin," Opt. Lett. 25, 1355 (2000), J.F. de Boer et al.. Birefringence imaging in biological tissue using polarization-sensitive optical coherence tomography. United States Patent 6,208,415, March 27, 2001, B.H. Park et al., "In vivo burn depth determination by high-speed fiber-based polarization sensitive optical coherence tomography," J. Biomed. Opt. 6, 474 (2001), M.C. Pierce et al., "Simultaneous intensity, birefringence, and flow measurements with high speed fiber-based optical coherence tomography," Opt. Lett. 27, 1534 (2002), and B.H. Park et al., "Real-time multi-functional optical coherence tomography," Opt. Exp. 11, 782 (2003). This can provide the polarization information which may be extracted, and further, maximize the effect of birefringence on a particular incident polarization state if parallel to the optic axis of the sample. It should be noted that this is not necessary for the exemplary embodiments of the present invention, e.g., so long as the two incident polarization states are unique for determination of diattenuation, and nonparallel in a Poincaré sphere representation if birefringence is one of the desired parameters.

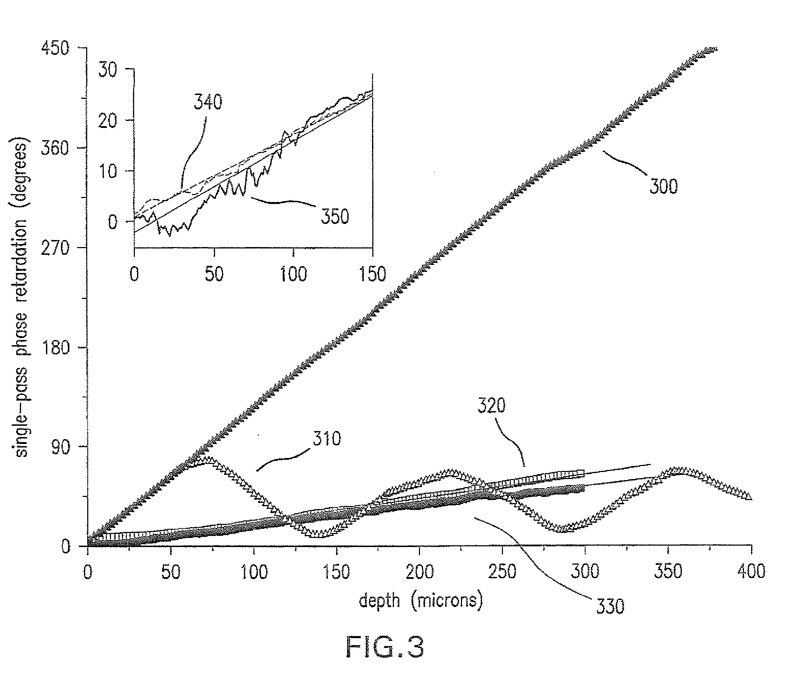

For example, Fig. 3 shows a plot of single-pass phase retardation as a function of depth. In Fig. 3, the light triangles and squares represent phase retardation values of chicken tendon and muscle, respectively, derived from PS-OCT images using a previously established analysis based on rotations in a Poincaré sphere representation. In particular, the images were acquired of exemplary chicken tendon and muscle samples. Data was analyzed with the presented method and a previously presented vector-based method, and shown in Fig. 3. With the conventional vector-based analysis technique, double-pass phase retardation has been restricted to values between 0 and $\pi$ radians. However, phase retardations calculated with the Jones matrix based approach can span for a full $2\pi$ range. This enables a determination of unwrappable phase retardation values in excess of $2\pi$ radians, as shown in the phase retardation plot for the tendon data 300 in Figure 3. The slopes of the phase retardation plots, e.g., 179.7°/mm for muscle 330 and 1184.4°/mm for tendon 300, are well within the expected parameters in accordance with the exemplary embodiment of the present invention. These values are similar to those calculated with the vector-based method, which yielded slopes of, e.g., 211.9°/mm and 1212.5°/mm for muscle 320 and tendon 310, respectively.

The analysis has been applied to an image of the superior region of the retinal nerve fiber layer (RNFL) acquired *in vivo* with a slit-lamp-adapted PS-OCT system as described in Cense *et al.* After averaging 10 points in depth, single-pass phase retardations as a function of depth 350 for RNFL as determined by the exemplary embodiment of the method according to the present invention, as well as the vector-based method 340 are displayed in the inset of Fig. 3. Linear-least-squares can fit over the full thickness of the RNFL yielded single-pass phase retardation slopes of 178.4°$\pm$1.3°/mm and 159.4°$\pm$1.4%mm using the two methods. For example, the dark triangles and squares are diattenuation values derived from the same PS-OCT images using the Jones matrix based analysis presented. Inset can be the same types of plots for data acquired from the superior region of the retinal nerve fiber layer of a human volunteer. Linear least-squares fits are shown for all plots.

Another exemplary implementation of the present invention is as follows. In a biological tissue, it if often the case that birefringence has a much greater effect on the polarization state of light than docs diattenuation. As such, it can be desirable to eliminate diattenuation from consideration, as small amounts of either effect can be interpreted as the other. In other words, a small amount of birefringence could easily be attributed to diattenuation and vice versa. In cases when it is known that diattenuation is negligible, the formulation in $Eq$. 1 can be modified simply by assuming that $P_1$ and $P_2$ are equal ($P_1 = P_2$). One method of determining the remaining polarization parameters is to start by normalizing the magnitudes of the complex electric fields and optimizing parameters $\alpha$, $\phi$, and $\theta$ to not only minimize the sum of the magnitudes of the off-diagonal elements, but also to minimize the difference between the magnitudes of the diagonal elements to match the condition that $P_1 = P_2$. The degree of phase retardation can then be extracted through the phase difference of the resulting diagonal elements, and the error estimated by some measure of the sum of the magnitudes of the off-diagonal elements and the difference of the magnitudes of the diagonal elements. In this case, Eq.1 can be used to not only compare the states reflected from the surface to those reflected from any depth to those from any other depth. For example, if all depths are compared to those a small distance above or below, the resulting polarization parameters may reflect the local properties of the tissue between the two points of comparison.

Another condition that may arise is that the birefringence should be ignored in favor of a use of only diattenuation. In this case, the parameter $\eta$ can be set to zero, and again, the parameters $\alpha$, $\phi$, and $\theta$ can be optimized to fit an appropriate condition. One such condition can be to minimize the imaginary portions of the diagonal elements simultaneously with the difference in magnitudes between the off-diagonal components. Alternatively, it may be desirable to place conditions on the orientation of the optic axis. In general, the formulation provided allows for selective determination of any and all non-depolarizing polarization parameters with a simple algorithm composed of optimizing the right hand side of Eq. 1 according to conditions appropriate for the situation, followed by extracting the desired polarization parameters from the remaining elements. This optimization can use any general functions of the complex electric fields of the detected light, including but not limited to their magnitudes, phases, and polynomial, logarithmic/exponential, trigonometric combinations thereof. Further, the use of incident polarization states perpendicular in a Poincaré sphere representation insures optimal detection of the sample polarization effects.

The methodology can be generalized as follows. Assume the Jones matrix of the sample, $J_S$, to be such that

$$J_T = J_{out} J_s J_{out}^{-1} = J_U J_S' J_U^{-1},$$ where $J_S'$ represents that portion of $\mathbf{J_S}$ that cannot be multiplied into $J_{out}$ to form $\mathbf{J_U}$. The polarization parameters of interest should then be isolated to within $\mathbf{J_S}'$. In this case, Eq. 1 generalizes to the form

$$e^{j\Delta\psi_1}\mathbf{J}_S' = \mathbf{J}_U^{-1}\begin{bmatrix} H_1' & H_2' \\ V_1' & V_2' \end{bmatrix}\begin{bmatrix} H_1 & e^{j\alpha}H_2 \\ V_1 & e^{j\alpha}V_2 \end{bmatrix}^{-1}\mathbf{J}_U$$

With knowledge of the form of $\mathbf{J_S}'$, it is possible to derive some appropriate function to determine the parameters, $\alpha$, $\Delta\psi$, and those used for $\mathbf{J_U}$, to best equate the two sides of the above equation. This function can include, but is not limited to, linear, polynomial, logarithmic, exponential, and trigonometric functions of magnitude and phase of the complex electric fields. Once this is accomplished, the polarization parameters of interest can be extracted from $\mathbf{J_S}'$.

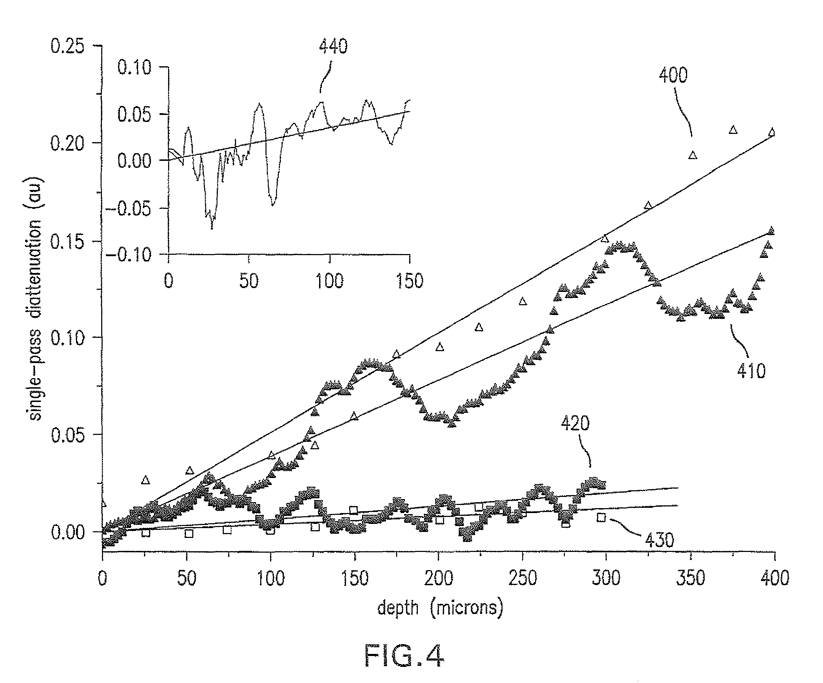

Fig. 4 shows a plot of a single-pass diattenuation as a function of depth obtained using the exemplary system and process according to the present invention. As a control measurement for diattenuation, a series of OCT images with varying single linear incident polarization states were acquired from the same locations of chicken tendon and muscle samples. The orientations for which the reflected polarization states from within the tissue varied minimally as a function of depth were chosen as those were the incident state which was aligned parallel or orthogonal to the sample optic axis. The corresponding intensity profiles described the attenuation parameters $P_1$ and $P_2$, from which depth-resolved control diattenuation plots were derived.

The resulting single-pass diattenuation plots for the PS-OCT and control measurements for tendon (labeled as 410 and 400, respectively) and muscle (labeled as 430 and 420, respectively) are shown in Fig. 4. A numerical simulation indicates that the average angular displacement of a state on the Poincaré sphere for a relatively small diattenuation $d$ is approximately $(40d)°$. For example, a diattenuation value of 0.20 can result in an average angular displacement in a Poincaré sphere representation of 8°. Given that a standard deviation on the order of 5° for individual polarization states reflected from the surface was found, the control and PS-OCT-derived diattenuation per unit depth of chicken muscle, $0.0380\pm0.0036$/mm versus $0.0662\pm0.0533$/mm, and tendon, $0.5027\pm0.353$/mm versus $0.3915\pm0.0365$/mm, reasonably or approximately agree.

The diattenuation in the same RNFL data previously utilized is determined and displayed as a function of depth in the inset (labeled as 440) in Fig. 4. Linear-least-squares fitting of the diattenuation values over the full RNFL thickness yielded a single-pass diattenuation per unit depth of $0.3543\pm0.1336$/mm.

In Fig. 4, the light triangles and squares represent control diattenuation values of chicken tendon and muscle, respectively, calculated from comparison of the reflectivity profiles for linear incident polarization states along and orthogonal to the fiber direction. The dark triangles and squares are diattenuation values derived from PS-OCT images acquired from the same tissues. Inset is a plot of the single-pass diattenuation derived from PS-OCT images acquired from the superior region of the retinal nerve fiber layer of a human volunteer. Linear least-squares fits can be shown for all plots.

An example of where such generalization can be useful is in the characterization of a rotating fiber probe. In this case, instead of comparing the reflected polarization states from the surface and from some depth within a sample, it is possible to compare the reflected polarization states from the end of the probe for two different rotation angles.

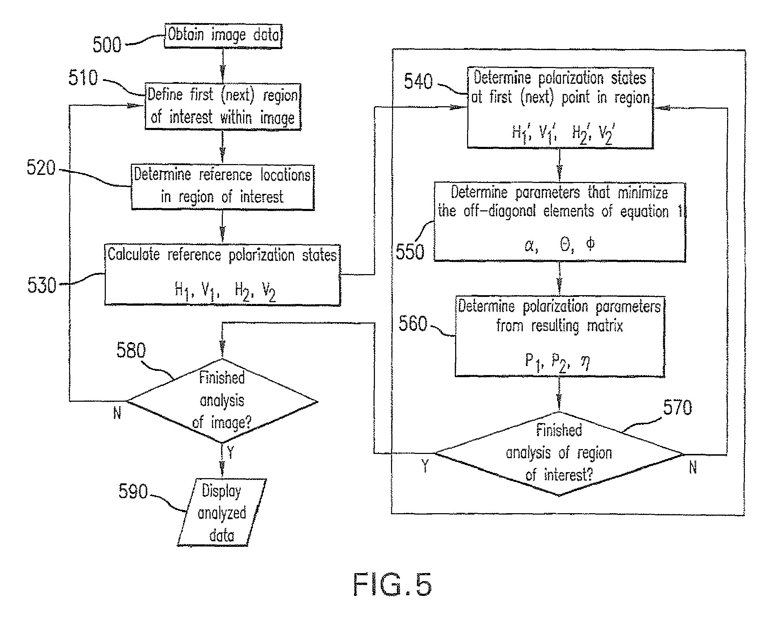

Fig. 5 shows a flow diagram of an exemplary embodiment of a method according to the present invention. As described herein above, the exemplary method can determine the non-depolarizing polarization properties of a region between two points. These points shall be referred to below as a reference point (i.e., different from the reference arm of the interferometer) and depth point.

In particular, the polarization state reflected from all points (e.g., determined by phase-sensitive measurement on two orthogonal detection channels) can be measured for at least two unique incident polarization states (step 500). These phase-sensitive (e.g., complex) polarization state measurements can be defined for any point,p, within the data set as $H_1(p)$, $V_1(p)$, $H_2(p)$, and $V_2(p)$. In step 510, a region of interest can be defined within the overall data set to be all depth points, and the polarization states thereof can be compared with those at a particular reference point in step 520. In step 530, the polarization states at the reference point can be determined and defined by, e.g., the quantities, $H_1$, $V_1$, $H_2$, and $V_2$, where the subscripts 1 and 2 generally refer to the two unique incident polarization states. For example, a single set of reference polarization states can be applicable for an entire image. The reference polarization states may be those reflected at, or near, the surface of the sample being imaged. In such case, a single region of interest exists, and the reference polarization states can be determined by averaging the polarization states from the surface of the entire image (to reduce noise effects).

When a rotating endoscopic probe is used, as the endoscope rotates, a fiber birefringence may be constantly changing, and thus will likely result in, e.g., a constantly changing set of polarization states reflected from the surface for various pairs of depth profiles. In this case, the entire image is used as a single region of interest which can lead to error. A region of interest may be defined by a small number of depth profile pairs, where the polarization states reflected from near the sample can be averaged. The result of this stage can be to define the region of interest within the entire image, and determine a set of reference polarization states, $H_1$, $V_1$, $H_2$, and $V_2$, that may apply to the region of interest.

Further, the polarization states are compared for all depth points within the region of interest to the reference polarization states. For example, the polarization states at the particular depth point may be determined in step 540, and may be defined by $H_1'$, $V_1'$, $H_2'$, $V_2'$. The parameters, e.g., $\alpha$, $\theta$, $\phi$, that are used for minimizing the off-diagonal elements of Eq. 1 using these values (depth point and reference polarization states) can then be determined in step 550. In step 560, the resulting approximately diagonal matrix provides the amounts of birefringence and diattenuation. This exemplary method can be repeated for all points within this region of interest by determining whether the analysis of the region of interest has been completed in step 570. If not, the process returns to step 540. Otherwise, the process continues to step 580. In particular, the determination is continued until all regions of interest within the entire image are analyzed by determining whether thee analysis of all images has been completed in step 580. If not, the process returns to step 510. Otherwise, the analyzed data is displayed in step 590.

The present invention can be used, e.g., when the polarization state reflected from a sample are detected or determined for at least two unique incident polarization states. The exemplary embodiment of the present invention can be used for data obtained from arrangements with reflective or transmissive reference delay lines for exemplary time-domain OCT, spectral-domain OCT, and OFDI techniques. The information from the two unique incident polarization states does not have necessarily have to be collected in the manner described above either; the only preference would be for light to be detected from a particular volume probed with both incident states. The present invention is valid and can be applied to determine the non-depolarizing polarization parameters for a region between two points within a sample, e.g., when the complex electric fields H and V can be determined, up to an overall phase, for both points and for two unique incident polarization states.

The foregoing merely illustrates the principles of the invention. Various modifications and alterations to the described embodiments will be apparent to those skilled in the art in view of the teachings herein. For example, the invention described herein is usable with the exemplary methods, systems and apparatus described in U.S. Provisional Patent Appn. No. 60/514,769 filed October 27, 2003, U.S. Patent Application Serial No. 60/599,809 filed on August 6, 2004 and International Patent Application No. PCT/US03/02349 filed on January 24, 2003, the disclosures of which are incorporated by reference herein in their entireties. It will thus be appreciated that those skilled in the art will be able to devise numerous systems, arrangements and methods which, although not explicitly shown or described herein, embody the principles of the invention and are thus within the spirit and scope of the present invention. In addition, all publications, patents and patent applications referenced above are incorporated herein by reference in their entireties.

Embodiments of the invention may have one or more features from the clauses that follow.

Clause 1. An arrangement for compensating for a polarization effect for a interferometric signal received from sample in an optical coherence tomography ("OCT") system, comprising:

a processing arrangement, which when executing a predetermined technique, is configured to:

(a) receive an interferometric information associated with the sample and a reference, and
(b) process the interferometric information thereby reducing a polarization effect created by a detection section of the OCT system on the interferometric signal, and determining an amount of a diattenuation of the sample, wherein the interferometric information is provided at least partially along at least one optical fiber which is provided in optical communication with and upstream from a polarization separating arrangement.

Clause 2. The arrangement according to clause 1, wherein the processing arrangement, when executing the predetermined technique, is further configured to determine at least one polarization property of the sample.

Clause 3. The arrangement according to clause 2, wherein the at least one polarization property includes a depolarizing property.

Clause 4. The arrangement according to clause 2, wherein the at least one polarization property includes a birefringence property.

Clause 5. The arrangement according to clause 2, wherein the at least one polarization property includes an optic axis of the at least one polarization property.

Clause 6. The arrangement according to clause 1, wherein the interferometric information includes further information associated with at least two polarization states incident on the sample.

Clause 7. The arrangement according to clause 2, wherein the interferometric information is processed by:

(g) determining a first state of one of the polarization states at a first location at least one of within or in a proximity of the sample,
(h) determining a second state of another one of the polarization states at least one of at or near the first location at least one of within or in the proximity of the sample,
(i) determining a third state of one of the polarization states at a second location at least one of within or in the proximity of the sample,
(j) determining a fourth state of another one of the polarization states at least one of at or near the second location at least one of within or in the proximity of the sample,
(k) generating a complex 2x2 matrix so as to transform the first and second states into the third and fourth states, respectively, and (1) decompose the matrix into a product of further matrixes, wherein first and second of the further matrices are unitary and inverse of one another, and selected to minimize off-diagonal elements of a third one of the further matrices.

Clause 8. The arrangement according to clause 7, wherein at least two of the first through fourth states which are obtained at locations that are at least one of the same as or different from the first and second locations are averaged.

Clause 9. A method for compensating for a polarization effect for a interferometric signal received from sample in an optical coherence tomography ("OCT") system, comprising:

receiving an interferometric information associated with the sample and a reference;
processing the interferometric information thereby reducing a polarization effect created by a detection section of the OCT system on the interferometric signal; and
determining an amount of a diattenuation of the sample, wherein the interferometric information is provided at least partially along at least one optical fiber which is provided in optical communication with and upstream from a polarization separating arrangement.

Clause 10. The method according to clause 9, wherein the interferometric information includes further information associated with at least two polarization states incident on the sample.

Clause 11. An apparatus comprising:

at least one first arrangement configured to provide at least one first electro-magnetic radiation, wherein a frequency of radiation provided by the at least one first arrangement varies over time;
at least one polarization modulating second arrangement configured to control polarization state of at least one first electro-magnetic radiation so as to produce at least one second electro-magnetic radiation; and
at least one third arrangement configured to receive the at least one second electro-magnetic radiation and provide at least one third electro-magnetic radiation to a sample and at least one fourth electro-magnetic radiation to a reference, wherein the third and fourth electro-magnetic radiations are associated with the at least one second electro-magnetic radiation.

Clause 12. The apparatus according to clause 11, wherein at least one fifth electro-magnetic radiation is provided from the sample, and at least one sixth electro-magnetic radiation is provided from the reference, wherein the fifth and sixth electro-magnetic radiations are associated with the third and fourth electro-magnetic radiations, respectively, further comprising:

at least one fourth arrangement configured to receive at least one seventh electromagnetic radiation which is associated with the fifth and sixth electro-magnetic radiations, and produce at least one eighth electromagnetic radiation having a first polarization state and at least one ninth electromagnetic radiation a second polarization state based on the at least one seventh electromagnetic radiation, wherein the first and second polarization states are different from one another.

Clause 13. The apparatus according to clause 12, further comprising:

at least one fifth arrangement configured to:

(i) at least one of receive or detect the eighth and ninth electromagnetic radiations, and determine at least one of an amplitude or a phase of at least one of the eighth and ninth electromagnetic radiations, or
(ii) at least one of receive or detect the eighth and ninth electromagnetic radiations, receive or detect at least one tenth radiation associated with at least one of the first, second, fourth or sixth electromagnetic radiations thereby reducing noise associated with fluctuations of at least one of the at least one first electromagnetic radiation or the at least one second electromagnetic radiation, and determine at least one of an amplitude or a phase of at least one of the at least one eighth electromagnetic radiation or the at least one ninth electromagnetic radiation.

Clause 14. The apparatus according to clause 13, wherein polarization states associated with the at least one fifth electromagnetic radiation are determined at different depth in at least one of the sample or a proximity of the sample as a function of the at least one of the amplitude or the phase of the at least one of the eighth and ninth electromagnetic radiations and based on the at least one second electro-magnetic radiation.

Clause 15. The apparatus according to clause 14, wherein the at least one of first through ninth electromagnetic radiations are propagated via at least one optical fiber.

Clause 16. The apparatus according to clause 11, further comprising:

at least one ophthalmic imaging sixth arrangement configured to received the at least one third electromagnetic radiation, and to produce the at least one fifth electromagnetic radiation.

Clause 17. The apparatus according to clause 14, further comprising:

a processing arrangement, which when executing a predetermined technique, is configured to:

(a) receive data associated with the at least one of the amplitude or the phase of the at least one of the eighth and ninth electromagnetic radiations, and
(b) process the data thereby reducing a polarization effect created by at least one portion of the apparatus on the at least one seventh electromagnetic radiation, and determining polarization properties of the sample.

Clause 18. The apparatus according to clause 17, wherein the polarization properties include at least one of birefringence, diattenuation, depolarization, optic axis of the birefringence, or optic axis of the diattenuation.

Clause 19. The arrangement according to claim 14, wherein the data is processed by:

(i) determining a first state of one of the polarization states at a first location at least one of within or in a proximity of the sample,
(ii) determining a second state of another one of the polarization states at least one of at or near the first location at least one of within or in the proximity of the sample,
(iii) determining a third state of one of the polarization states at a second location at least one of within or in the proximity of the sample,
(iv) determining a fourth state of another one of the polarization states at least one of at or near the second location at least one of within or in the proximity of the sample,
(v) generating a complex 2x2 matrix so as to transform the first and second states into the third and fourth states, respectively, and
(vi) decompose the matrix into a product of further matrixes, wherein first and second of the further matrices are unitary and inverse of one another, and selected to minimize off-diagonal elements of a third one of the further matrices.

Clause 20. The arrangement according to clause 19, wherein at least two of the first through fourth states which are obtained at locations that are at least one of the same as or different from the first and second locations are averaged.

Clause 21. A method for providing electromagnetic radiation to a sample, comprising:

providing at least one first electro-magnetic radiation, wherein a frequency of radiation provided by the at least one first arrangement varies over time;
controlling a polarization state of at least one first electro-magnetic radiation using at least one polarization modulating arrangement so as to produce at least one second electro-magnetic radiation; and
receiving the at least one second electro-magnetic radiation and providing at least one third electro-magnetic radiation to the sample and at least one fourth electro-magnetic radiation to a reference, wherein the third and fourth electro-magnetic radiations are associated with at least one second electro-magnetic radiation.

The protection sought is recited in the claims that follow.

**Claims**

1. An apparatus comprising:

   at least one first arrangement configured to provide at least one first electro-magnetic radiation, wherein a frequency of radiation provided by the at least one first arrangement varies over time;
   at least one polarization modulating second arrangement configured to control a polarization state of the at least one first electro-magnetic radiation so as to produce at least one second electro-magnetic radiation; and
   at least one third non-diattenuating arrangement configured to receive the at least one second electro-magnetic radiation and provide at least one third electro-magnetic radiation to a sample and at least one fourth electro-magnetic radiation to a reference, wherein the third and fourth electro-magnetic radiations are associated with the at least one second electro-magnetic radiation.

2. The apparatus according to claim 1, wherein at least one fifth electro-magnetic radiation is provided from the sample, and at least one sixth electro-magnetic radiation is provided from the reference, wherein the fifth and sixth electro-magnetic radiations are associated with the third and fourth electro-magnetic radiations, respectively, further comprising: at least one fourth arrangement configured to receive at least one seventh electromagnetic radiation which is associated with the fifth and sixth electro-magnetic radiations, and produce at least one eighth electromagnetic radiation having a first polarization state and at least one ninth electromagnetic radiation a second polarization state based on the at least one seventh electromagnetic radiation, wherein the first and second polarization states are different from one another.

3. The apparatus according to claim 2, further comprising:

   at least one fifth arrangement configured to:

      i. at least one of receive or detect the eighth and ninth electromagnetic radiations, and determine at least one of an amplitude or a phase of at least one of the eighth and ninth electromagnetic radiations, or
      ii. at least one of receive or detect the eighth and ninth electromagnetic radiations, receive or detect at least one tenth radiation associated with at least one of the first, second, fourth or sixth electromagnetic radiations thereby reducing noise associated with fluctuations of at least one of the at least one first electromagnetic radiation or the at least one second electromagnetic radiation, and determine at least one of an amplitude or a phase of at least one of the at least one eighth electromagnetic radiation or the at least one ninth electromagnetic radiation.

4. The apparatus according to claim 3, wherein polarization states associated with the at least one fifth electromagnetic radiation are determined at different depths in at least one of the sample or a proximity of the sample as a function of the phase of the at least one of the eighth and ninth electromagnetic radiations and based on the at least one second electro-magnetic radiation.

5. The apparatus according to claim 4, wherein the at least one of first through ninth electromagnetic radiations are propagated via at least one optical fiber.

**6.** The apparatus according to claim 1, further comprising:

at least one ophthalmic imaging arrangement configured to received the at least one third electromagnetic radiation, and to produce the at least one fifth electromagnetic radiation, the at least one ophthalmic imaging arrangement is specifically configured to image at least one portion of an eye which is the sample.

**7.** The apparatus according to claim 4, further comprising:

a processing arrangement, which when executing a predetermined technique, is specifically configured to:

a) receive data associated with the phase of the at least one of the eighth and ninth electromagnetic radiations, and
b) process the data thereby reducing a polarization effect created by at least one portion of the apparatus on the at least one seventh electromagnetic radiation, and determining polarization properties of the sample.

**8.** The apparatus according to claim 7, wherein the polarization properties include at least one of birefringence, diattenuation, depolarization, optic axis of the birefringence, or optic axis of the diattenuation.

**9.** The arrangement according to claim 4, wherein the processing arrangement is configured to process the data by:

i. determining a first state of one of the polarization states at a first location at least one of within or in a proximity of the sample,
ii. determining a second state of another one of the polarization states at least one of at or near the first location at least one of within or in the proximity of the sample,
iii. determining a third state of one of the polarization states at a second location at least one of within or in the proximity of the sample,
iv. determining a fourth state of another one of the polarization states at least one of at or near the second location at least one of within or in the proximity of the sample,
v. generating a complex 2x2 matrix so as to transform the first and second states into the third and fourth states, respectively, and
vi. <u>decomposing</u> the matrix into a product of further matrixes, wherein first and second of the further matrices are unitary and inverse of one another, and selected to minimize off-dlelgonal elements of a third one of the further matrices.

**10.** The arrangement according to claim 9, wherein at least two of the first through fourth states which are obtained at locations that are at least one of the same as or different from the first and second locations are averaged.

**11.** The arrangement according to claim 1, wherein the at least one polarization modulating second arrangement is further configured to selectively modulate the at least one second electro-magnetic radiation to be in at least one of a first polarization state or a second polarization state.

**12.** The arrangement according to claim 2, wherein the at least one third electro-magnetic radiation has a third polarization state and the at least one fourth electro-magnetic radiation has a fourth polarization state, and wherein the third and fourth polarization states are utilized to produce the at least one modulated state of the first polarization state or the second polarization state.

**13.** The arrangement according to claim 3, wherein the eighth and ninth electromagnetic radiations have respective polarization states which are different from one another.

**14.** The arrangement according to claim 14, wherein the polarization states associated with the at least one fifth electromagnetic radiation are determined at the different depth in the at least one of the sample or the proximity of the sample as a further function of the amplitude of the at least one of the eighth and ninth electromagnetic radiations.

**15.** An apparatus comprising:

at least one first arrangement configured to provide at least one first electro-magnetic radiation, wherein a frequency of radiation provided by the at least one first arrangement varies over time;
at least one second arrangement configured to produce at least one second electro-magnetic radiation based on the at least one first electromagnetic radiation and to selectively modulate the at least one second electromagnetic radiation to be in at least one of a first polarization state or a second polarization state; and at least one third arrangement configured to receive the at least one second electro-magnetic radiation and provide at least one third electromagnetic radiation to a sample and at least one fourth electro-magnetic radiation to a reference, wherein the third and fourth electro-magnetic radiations are associated with the at least one second electro-magnetic radiation.

16. The arrangement according to claim 26, wherein the at least one third electro-magnetic radiation has a third polarization state and the at least one fourth electro-magnetic radiation has a fourth polarization state, and wherein the third and fourth polarization states are utilized to produce the at least one modulated state of the first polarization state or the polarization second state.

FIG.1

EP 2 272 424 A1

FIG.2

FIG.3

EP 2 272 424 A1

FIG.4

FIG.5

Europäisches
Patentamt

European
Patent Office

Office européen
des brevets

# EUROPEAN SEARCH REPORT

Application Number

EP 10 18 5570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | JUN ZHANG ET AL: "Full range polarization-sensitive Fourier domain optical coherence tomography", OPTICS EXPRESS, OSA (OPTICAL SOCIETY OF AMERICA), WASHINGTON DC, (US), vol. 12, no. 24, 29 November 2004 (2004-11-29), pages 6033-6039, XP002415697, ISSN: 1094-4087, DOI: DOI:10.1364/OPEX.12.006033 * page 6034, paragraph 1 - page 6035, paragraph 1 * * page 6037, paragraph 2 * * figure 1 * | 1-8, 11-16 | INV. A61B5/00 G01N21/47 G01B9/02 |
| Y,P | PARK B H ET AL: "Jones matrix analysis for a polarization-sensitive optical coherence tomography system using fiber-optic components", OPTICS LETTERS OPT. SOC. AMERICA USA, vol. 29, no. 21, 1 November 2004 (2004-11-01), pages 2512-2514, XP002375104, ISSN: 0146-9592 * the whole document * | 1-8, 11-16 | |
| Y | YUN S H ET AL: "High-speed optical frequency-domain imaging", OPTICS EXPRESS, OSA (OPTICAL SOCIETY OF AMERICA), WASHINGTON DC, (US), vol. 11, no. 22, 3 November 2003 (2003-11-03), pages 2923-2963, XP008083922, ISSN: 1094-4087, DOI: DOI:10.1364/OE.11.002953 * page 2954, paragraph 1 - page 2955, paragraph 1 * * figure 1 * | 1-8, 11-16 | TECHNICAL FIELDS SEARCHED (IPC) A61B G01N G01B |

-/--

The present search report has been drawn up for all claims

| Place of search | Date of completion of the search | Examiner |
|---|---|---|
| Berlin | 24 November 2010 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 18 5570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YASUNO YOSHIAKI ET AL: "Polarization-sensitive complex Fourier domain optical coherence tomography for Jones matrix imaging of biological samples", APPLIED PHYSICS LETTERS, AIP, AMERICAN INSTITUTE OF PHYSICS, MELVILLE, NY, US, vol. 85, no. 15, 1 January 2004 (2004-01-01), pages 3023-3025, XP012062847, ISSN: 0003-6951, DOI: DOI:10.1063/1.1804233 * page 3023, left-hand column, paragraph 1 - page 3024, left-hand column, paragraph 3 * * page 3024, right-hand column, paragraphs 3,4 * * page 3025, right-hand column, paragraph 3 * * figure 1 * | 1-8, 11-16 | |
| A,D | SAXER C E ET AL: "High-speed fiber-based polarization-sensitive optical coherence tomography of in vivo human skin", OPTICS LETTERS OPT. SOC. AMERICA USA, vol. 25, no. 18, 15 September 2000 (2000-09-15), pages 1355-1357, XP002375101, ISSN: 0146-9592 * page 1355, left-hand column, paragraph 2 - page 1357, right-hand column, paragraph 2 * | 1-8, 11-16 | TECHNICAL FIELDS SEARCHED (IPC) |

-/--

The present search report has been drawn up for all claims

| Place of search | Date of completion of the search | Examiner |
|---|---|---|
| Berlin | 24 November 2010 | Völlinger, Martin |

EPO FORM 1503 03.82 (P04C01)

| | Europäisches Patentamt European Patent Office Office européen des brevets | EUROPEAN SEARCH REPORT | Application Number EP 10 18 5570 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | SHULIANG JIAO ET AL: "Two-dimensional depth-resolved Mueller matrix of biological tissue measured with double-beam polarization-sensitive optical coherence tomography", OPTICS LETTERS OPT. SOC. AMERICA USA, vol. 27, no. 2, 15 January 2002 (2002-01-15), pages 101-103, XP002375103, ISSN: 0146-9592 * page 101, left-hand column, paragraph 2 - page 101, right-hand column, paragraph 1 * * page 102, left-hand column, paragraphs 2,3 * * page 102, right-hand column, paragraph 4 - page 103, right-hand column, paragraph 2 * ----- | 1-8, 11-16 | |
| A,D | SHULIANG JIAO ET AL: "Optical-fiber-based Mueller optical coherence tomography", OPTICS LETTERS OPT. SOC. AMERICA USA, vol. 28, no. 14, 15 July 2003 (2003-07-15), pages 1206-1208, XP002375102, ISSN: 0146-9592 * the whole document * ----- | 1-8, 11-16 | TECHNICAL FIELDS SEARCHED (IPC) |
| A | PIERCE M C ET AL: "Simultaneous intensity, birefringence, and flow measurements with high-speed fiber-based optical coherence tomography", OPTICS LETTERS OPT. SOC. AMERICA USA, vol. 27, no. 17, 1 September 2002 (2002-09-01), pages 1534-1536, XP002375105, ISSN: 0146-9592 * page 1534, left-hand column, paragraph 4 - page 1535, left-hand column, paragraph 1 * ----- | 1,15 | |

The present search report has been drawn up for all claims

| Place of search | Date of completion of the search | Examiner |
|---|---|---|
| Berlin | 24 November 2010 | Völlinger, Martin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

**Patent documents cited in the description**

- US 62377304 P
- US 0302349 W
- US 514769 P
- US 6208415 B
- US 51476903 P
- US 59980904 P

**Non-patent literature cited in the description**

- **Huang et al.** Optical Coherence Tomography. *Science,* 1991, vol. 254, 1178
- **Saxer et al.** High-speed fiber-based polarization-sensitive optical coherence tomography of in vivo human skin. *Opt. Lett.,* 2000, vol. 25, 1355
- **J.F. de Boer et al.** Determination of the depth-resolved Stokes parameters of light backscattered from turbid media by use of polarization-sensitive optical coherence tomography. *Opt. Lett.,* 1999, vol. 24, 300
- **B. Cense et al.** In vivo depth-resolved birefringence measurements of the human retinal nerve fiber layer by polarization-sensitive optical coherence tomography. *Opt. Lett.,* 2002, vol. 27, 1610
- **B.H. Park et al.** In vivo burn depth determination by high-speed fiber-based polarization sensitive optical coherence tomography. *J. Biomed. Opt.,* 2001, vol. 6, 474
- **S.L. Jiao et al.** Two-dimensional depth-resolved Mueller matrix of biological tissue measured with double-beam polarization-sensitive optical coherence tomography. *Opt. Lett.,* 2002, vol. 27, 101
- **S. Jiao et al.** Optical-fiber-based Mueller optical coherence tomography. *Opt. Lett.,* 2003, vol. 28, 1206
- **S.L. Jiao et al.** Depth-resolved two-dimensional Stokes vectors of backscattered light and Mueller matrices of biological tissue measured with optical coherence tomography. *Appl. Opt,* 2000, vol. 39, 6318
- **S.L. Jiao ; L.V. Wang.** Jones-matrix imaging of biological tissues with quadruple-channel optical coherence tomography. *J. Biomed. Opt,* 2002, vol. 7, 350
- **N.A. Nassif et al.** In vivo human retinal imaging by ultrahigh-speed spectral domain optical coherence tomography. *Opt. Lett.,* 2004, vol. 29, 480
- **S.H. Yun et al.** High-speed optical frequency-domain imaging. *Opt. Exp.,* 2003, vol. 11, 2953
- **J.J. Gil et al.** Obtainment of the polarizing and retardation parameters of a non-depolarizing optical system from the polar decomposition of its Mueller matrix. *Optik,* 1987, vol. 76, 67
- **B.H. Park et al.** Real-time multi-functional optical coherence tomography. *Opt. Exp.,* 2003, vol. 11, 782
- **C.E. Saxer et al.** High-speed fiber-based polarization-sensitive optical coherence tomography of in vivo human skin. *Opt. Lett.,* 2000, vol. 25, 1355
- **M.C. Pierce et al.** Simultaneous intensity, birefringence, and flow measurements with high speed fiber-based optical coherence tomography. *Opt. Lett.,* 2002, vol. 27, 1534